# EUROPEAN PATENT APPLICATION

(11) **EP 0 729 758 A2**
(43) Date of publication of application: **04.09.1996**
(21) Application number: 96300931.1
(22) Date of filing: 12.02.1996
(51) Int. Cl.: A61K 31/505

(54) **Pyrazolopyrimidines and pyrrolopyrimidines for treatment of neuronal and other disorders**

(30) Priority: 02.03.1995 US 397527
(71) Applicant: PFIZER INC., New York, N.Y. 10017 (US)
(72) Inventor: Yuhpyng, Chen L., Waterford, Connecticut 06385 (US)
(74) Representative: Hayles, James Richard

(57) **Abstract**

Pyrazolopyrimidines and pyrrolopyrimidines of the general formula 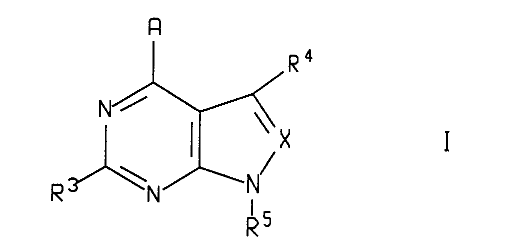
wherein A, X, R³, R⁴ and R⁵ are defined in the application, and pharmaceutically acceptable salts of such compounds, are useful in the treatment or prevention of certain neuronal and other disorders.

## Description

This invention relates to the use of pyrazolopyrimidines and pyrrolopyrimidines of the general formula 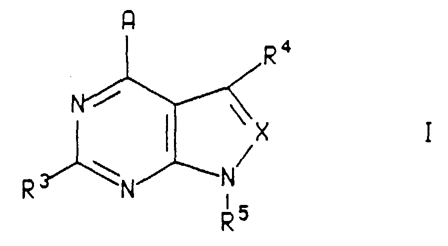 wherein A, X, R³, R⁴ and R⁵ are as defined below, and the pharmaceutically acceptable salts of such compounds, to treat or prevent certain neuronal and other disorders.

The compounds of the formula I above exhibit corticotropin-releasing factor (CRF) receptor antagonist activity and are useful in the treatment and prevention of head trauma, spinal cord trauma, ischemic neuronal damage (e.g., cerebral ischemia such as cerebral hippocampal ischemia), excitotoxic neuronal damage, epilepsy, stroke, stress induced immune dysfunctions, phobias, muscular spasms, Parkinson's disease, Huntington's disease, urinary incontinence, senile dementia of the Alzheimer's type, multiinfarct dementia, amyotrophic lateral sclerosis, chemical dependencies and addictions (e.g., dependencies on alcohol, cocaine, heroin, benzodiazepines, or other drugs), and hypoglycemia.

The compounds of formula I are also useful for treatment of stress induced diseases in various livestock animal species caused by transportation (shipment) conditions such as a severe confining environment, exposure to a novel environment, confrontation with unfamiliar animals, noise, movement, hunger or thirst (e.g., bovine shipping fever, pasteurellosis, porcine stress syndrome or equine paroxysmal atrial fibrillation).

Certain substituted pyrrolopyrimidines have been referred to in the scientific literature. U.S. Patent 4,229,453, which issued on October 21, 1980, refers to 4-amino substituted pyrrolopyrimidines for treating CNS illnesses or inflammations. Robins, Can. J. Chem., 55, 1251 (1977), refers to the antibiotic tubercidin having a 7-ribofuranosyl group attached to 4-aminopyrrolopyrimidine. German Patent Application 3145287, which was published on May 19, 1983, refers to three 7-bromophenyl-5,6-dimethylpyrrolopyrimidines as having analgesic, sedative, anti-convulsant and anti-inflammatory activity.

Certain substituted pyrazolopyrimidines have also been referred to in the literature. For instance, European Patent Application 496,617, which was published on July 29, 1992, refers to adenosine kinase inhibitors, among which are 1-ribofuranosylpyrazolopyrimidines and 1-(substituted ribofuranosyl)pyrazolopyrimidines. U.S. Patent No. 4,904,666, which issued on February 27, 1990, refers to pyrazolopyrimidines having 1-tetrahydrofuranyl or 1-tetrahydropyranyl substituents. Senga et at, J. Heterocyclic Chem., 19, 1565 (1982) refers to certain pyrazolopyrimidines having xanthine oxidase inhibitory activity. Other pyrazolopyrimidines are mentioned in U.S. Patent Nos. 2,965,643 and 3,600,389, which issued, respectively, on December 20, 1960 and August 17, 1971.

Compounds of the formula I wherein X is nitrogen and the pharmaceutically acceptable acid addition salts of such compounds, as well as methods for preparing such compounds and salts, are referred to in World Patent Application PCT/US 93/11333, which, was filed on November 26, 1993.

This World Patent Application also refers to the use of compounds of the formula I in the treatment of illnesses induced or facilitated by corticotropin releasing factor and in the treatment of inflammatory disorders such as arthritis, asthma and allergies, anxiety, depression, fatigue syndrome, headache, pain, cancer, irritable bowel syndrome, Crohn's disease, spastic colon, immune dysfunction, human immunodefiency virus (HIV) infections, neurodegenerative diseases such as Alzheimer's disease, gastrointestinal diseases, eating disorders such as anorexia nervosa, hemorrhagic stress, drug and alcohol withdrawal symptoms, drug addiction, stress-induced psychotic episodes and fertility problems.

Compounds of the formula I wherein X is CR⁶ and the pharmaceutically acceptable acid addition salts of such compounds, as well as methods of preparing such compounds and salts, are referred to in World Patent Aplication PCT/US 93/10715, which was filed on 12 November 1993. This World Patent Application also refers to the use of compounds of the formula I wherein X is CR⁶ in the treatment of illnesses induced or facilitated by corticotropin releasing factor and in the treatment of inflammatory disorders such as arthritis, asthma and allergies, anxiety, depression, fatigue syndrome, headache, pain, cancer, irritable bowel syndrome, Crohn's disease, spastic colon, immune dysfunction, human immunodeficiency virus (HIV) infections, neurodegenerative diseases such as Alzheimer's disease, gastrointestinal diseases, eating disorders such as anorexia nervosa, hemorrhagic stress, drug and alcohol withdrawal symptoms, drug addiction, stress-induced psychotic episodes, and fertility problems.

### Summary of the Invention

This invention relates to a method of treating, preventing or inhibiting a disorder selected from head traumas, spinal cord trauma, ischemic neuronal damage (e.g., cerebral ischemia such as cerebral hippocampal ischemia), excitotoxic neuronal damage, epilepsy, stroke, stress induced immune dysfunctions, (e.g., porcine stress syndrome, obsessive-compulsive disorder, bovine shipping fever, equine paroxysmal fibrillation, phobias and dysfunctions induced by confinement in chickens, sheering stress in sheep or human-animal interaction related stress in dogs), phobias, muscular spasms, Parkinson's disease, Huntington's disease, urinary incontinence, senile dementia of the Alzheimer's type, multiinfarct dementia, amyotrophic lateral sclerosis, chemical dependencies and addictions (e.g., dependencies on alcohol, cocaine, heroin, benzodiazepines, or other drugs), and hypoglycemia in a mammal, including a human, comprising administering to such mammal an amount of a compound of the formula 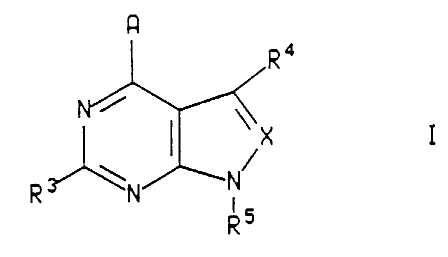 wherein
X is nitrogen or -CR⁶;
A is -NR¹R², -CR¹R²R¹¹, -C(=CR²R¹²)R¹,-NHCR¹R²R¹¹,-OCR¹R²R¹¹, -SCR¹R²R¹¹, -NHNR¹R², -CR²R¹¹NHR¹, -CR²R¹¹OR¹, -CR²R¹¹SR¹, or -C(O)R²;
R¹ is hydrogen, or C₁-C₆ alkyl which may optionally be substituted with from one to two substituents independently selected from the group consisting of hydroxy, fluoro, chloro, bromo, iodo, C₁-C₈ alkoxy, 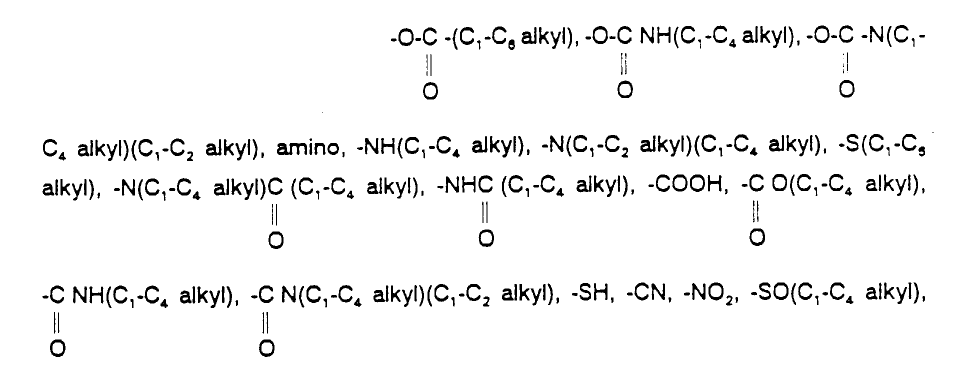 -SO₂(C₁-C₄ alkyl), -SO₂NH(C₁C₄ alkyl), -SO₂N(C₁-C₄ alkyl)(C₁-C₂ alkyl), and wherein each of the foregoing C₁-C₆ alkyl moieties in the definition of R¹ may contain one or two double or triple bonds;
R² is C₁-C₁₂ alkyl, aryl or -(C₁-C₁₀ alkylene)aryl wherein said aryl is phenyl, naphthyl, thienyl, benzothienyl, pyridyl, quinolyl, pyrazinyl, pyrimidyl, imidazolyl, furanyl, benzofuranyl, benzothiazolyl, isothiazolyl, benzisothiazolyl, thiazolyl, isoxazolyl, benzisoxazolyl, benzimidazolyl, triazolyl, pyrazolyl, pyrrolyl, indolyl, pyrrolopyridyl, oxazolyl, or benzoxazolyl; 3- to 8-membered cycloalkyl or -(C₁-C₆ alkylene) cycloalkyl, wherein one or two of the carbon atoms of any of said cycloalkyl moieties may optionally be replaced, independently, by O, S or N-Z wherein Z is hydrogen, C₁-C₄ alkyl or C₁-C₄ alkanoyl, and wherein R² may optionally be substituted with from one to three substituents independently selected from chloro, fluoro and C₁-C₄ alkyl, or by one substituent selected from hydroxy, bromo, iodo, C₁-C₆ alkoxy, 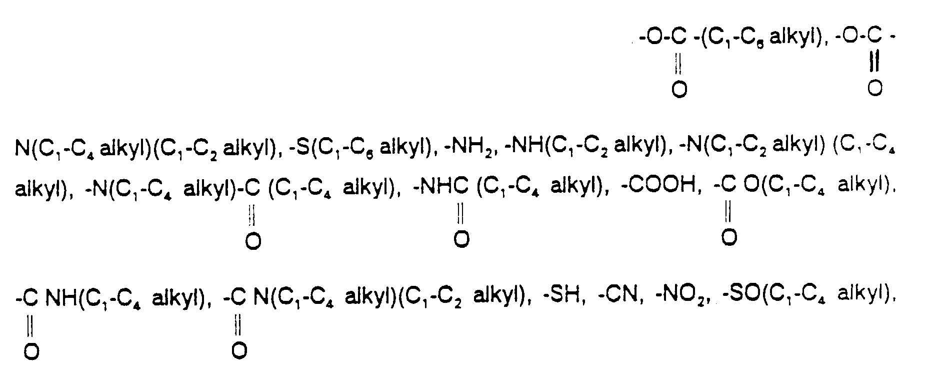 -SO₂(C₁-C₄ alkyl), -SO₂NH(C₁-C₄ alkyl), and -SO₂N(C₁-C₄ alkyl)(C₁-C₂ alkyl), and wherein each of the foregoing C₁-C₁₂ alkyl and C₁-C₁₀ alkylene moieties in the definition may optionally contain one to three double or triple bonds; or
R¹ and R², taken together with the atom to which they are attached, may form a saturated 3- to 8-membered ring which, if it is a 5- to 8-membered ring, may optionally contain one or two double bonds, and wherein one or two of the carbon atoms of said 5- to 8- membered ring may optionally be replaced, independently, by O, S or N-Z wherein Z is hydrogen, C₁-C₄ alkyl, C₁-C₄ alkanoyl or benzyl;
R³ is hydrogen, C₁-C₆ alkyl, fluoro, chloro, bromo, iodo, hydroxy, amino, -O(C₁-C₆ alkyl), -NH(C₁-C₆ alkyl), -N(C₁-C₄ alkyl)(C₁-C₂ alkyl), -SH, -S(C₁-C₄ alkyl), -SO(C₁-C₄ alkyl), or -SO₂(C₁-C₄ alkyl), wherein each of the foregoing C₁-C₄ alkyl and C₁-C₆ alkyl moieties in the definition of R³ may contain one double or triple bond and may optionally be substituted with from 1 to 3 substituents independently selected from the group consisting of hydroxy, C₁-C₃ alkoxy, fluoro, chloro and C₁-C₃ thioalkyl;
R⁴ is hydrogen, C₁-C₆ alkyl, fluoro, chloro, bromo, iodo, C₁-C₆ alkoxy, formyl, -NH(C₁-C₆ alkyl), -N(C₁-C₆ alkyl)(C₁-C₂ alkyl), -SOₙ(C₁-C₆ alkyl), wherein n is 0, 1 or 2, cyano, hydroxy, carboxy, or amido, wherein each of the foregoing C₁-C₆ alkyl moieties in the definition of R³ may optionally be substituted with one substituent selected from hydroxy, trifluoromethyl, amino, carboxy, amido, 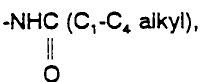 -NH(C₁-C₄ alkyl), -N(C₁-C₄ alkyl)(C₁-C₂ alkyl), 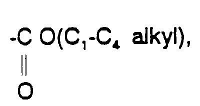 C₁-C₃ alkoxy, C₁-C₃ thioalkyl, fluoro, bromo, chloro, iodo, cyano and nitro;
R⁵ is phenyl, naphthyl, thienyl, benzothienyl, pyridyl, quinolyl, pyrazinyl, pyrimidyl, imidazolyl, furanyl, benzofuranyl, benzothiazolyl, isothiazolyl, benzoisothiazolyl, thiazolyl, isoxazolyl, benzisoxazolyl, benzimidazolyl, tritolyl, pyrazolyl, pyrrolyl, indolyl, pyrrolopyridyl, benzoxazolyl, oxazolyl, pyrrolidinyl, thiazolidinyl, morpholinyl, piperidinyl, piperazinyl, tetrazolyl, or a 3- to 8-membered cycloalkyl or 9- to 12-membered bicycloalkyl ring, wherein one or two of the carbon atoms in said ring may optionally be replaced, independently, by O, S or N-Z wherein Z is hydrogen, C₁-C₄ alkyl, C₁-C₄ alkanoyl, phenyl or benzyl, and wherein each of the above R⁵ groups may optionally be substituted with one or more substituents, preferably with two or three substituents, independently selected from fluoro, chloro, bromo, formyl, C₁-C₆ alkyl, C₁-C₆ alkoxy and trifluoromethyl, or with one substituent selected from hydroxy, iodo, cyano, nitro, amino, -NH(C₁-C₄ alkyl), -N(C₁-C₄ alkyl)(C₁-C₂ alkyl), -COO(C₁-C₄ alkyl), -CO(C₁-C₄ alkyl), -SO₂NH(C₁-C₄ alkyl), -SO₂N(C₁-C₄ alkyl)(C₁-C₂ alkyl), -SO₂NH₂, -NHSO₂(C₁-C₄ alkyl), -S(C₁-C₆ alkyl), -SO₂(C₁-C₆ alkyl), and wherein each of the foregoing C₁-C₄ alkyl and C₁-C₆ alkyl moieties in the definition of R⁵ may optionally be substituted with from one to two substituents independently selected from fluoro, chloro, hydroxy, C₁-C₄ alkoxy, amino, methylamino, dimethylamino and acetyl, and wherein each of the foregoing C₁-C₄ alkyl and C₁-C₆ alkyl moieties in the definition of R⁵ may optionally contain one double or triple bond; with the proviso that R⁵ is not unsubstituted phenyl;
R⁶ is hydrogen, C₁-C₆ alkyl, fluoro, chloro, bromo, iodo, C₁-C₆ alkoxy, formyl, amino, -NH(C₁-C₆ alkyl), N(C₁-C₆ alkyl)(C₁-C₂ alkyl), -SOₙ(C₁-C₆ alkyl), wherein n is 0, 1 or 2, cyano, carboxy, or amido, and wherein each of the foregoing (C₁-C₆)alkyl moieties in the definition of R⁶ may be optionally substituted with one substituent selected from hydroxy, trifluoromethyl, amino, carboxy, amido, 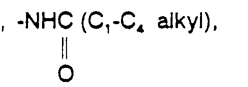 -NH(C₁-C₄ alkyl), -N(C₁-C₄ alkyl)(C₁-C₂ alkyl), 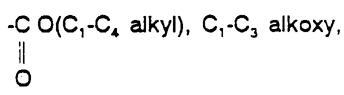 C₁-C₃ thioalkyl, fluoro, bromo, chloro, iodo, cyano and nitro;
R¹¹ is hydrogen, hydroxy, fluoro, chloro, -COO(C₁-C₂ alkyl), cyano, or -CO(C₁-C₂ alkyl); and
R¹² is hydrogen or C₁-C₄ alkyl;
with the proviso that: (1) when X is -CR⁶, A is not straight chain alkyl; (2) when X is CR⁶ and R⁵ is unsubstituted cycloalkyl and R³ and R⁴ are both hydrogen and R⁶ is hydrogen or methyl, then A is not NHR² wherein R² is benzyl or thienylmethyl, and (3) when X is -CR⁶ and R⁵ is p-bromophenyl and R³, R⁴ and R⁶ are methyl, then A is not methylamino or hydroxyethylamino;
and when X is nitrogen, with the further proviso that:
(a) A is not straight chain C₁-C₁₂ alkyl;
(b) R⁵ is not a sugar group;
(c) when R³ and R⁴ are hydrogen and R⁵ is chlorophenyl, then A is not -NH-CH(CH₃)-(CH₂)₃-N(C₂H₅)₂;
(d) when R³ and R⁴ are hydrogen and A is -NR¹ R² wherein R¹ is C₃-C₇ cycloalkyl, and R² is C₂-C₆ alkenyl, phenyl-(C₁-C₆ alkylene) or hetero-(C₁-C₆ alkylene) wherein the hetero radical is furyl, thienyl or pyridinyl, and wherein said phenyl may be substituted with one or more substituents independently selected from fluoro, chloro, bromo and iodo, then R⁵ is not tetrahydrofuranyl or tetrahydropyranyl;
(e) when R³ is methoxy, methylthio, or methylsulfonyl, R⁴ is hydrogen, and R⁵ is tetrahydrofuranyl or tetrahydropyranyl, then A is not -NH(C₁-C₂alkyl), morpholinyl, hydrazino, or -NHC₂H₄C₆H₅, the phenyl of which may be substituted by one methyl or two methoxy groups;
(f) when R³ is hydrogen, C₁-C₆ alkyl, chloro, bromo, -SH, or -S-(C₁-C₄ alkyl), R⁴ is hydrogen and R⁵ is C₃-C₈ cycloalkyl, then A is not hydrazino, -NH(C₁-C₂ alkyl) or -N(C₁-C₆ alkyl) (C₁-C₁₂ alkyl);
(g) when R³ and R⁴ are hydrogen and A is -NH(CH₂)ₘ COOH wherein m is 1-12, then R⁵ is not phenyl substituted by one fluoro, chloro, bromo or iodo group;
(h) when R³ is hydrogen, hydroxy, methylthio, chloro or -NHbenzyl, R⁴ is hydrogen, and R⁵ is chlorophenyl or bromophenyl, then A is not -NH(C₁-C₁₂ alkyl), -NHallyl, or -N(C₁-C₆ alkyl) (C₁-C₁₂ alkyl), wherein said C₁-C₁₂ alkyl may be substituted by -NC₂H₅, or -NH benzyl which may be substituted by one or two bromo, chloro, fluoro, -NC₂H₅ phenyl or morpholinopropyl groups;
(i) when R³ and R⁴ are hydrogen and R⁵ is nitrophenyl, then A is not -NHR² wherein R² is phenyl, benzyl or C₁-C₁₂ alkyl which may be substituted by one or two hydroxy groups;
(j) when R³ is chloro or -O(C₁-C₆ alkyl), R⁴ is hydrogen, and A is -NR¹R² wherein R¹ and R² are independently hydrogen or C₁-C₆ alkyl, then R⁵ is not chlorophenyl; and
(k) when R³ is hydrogen, A is benzyl or phenethyl, and R⁴ is fluoro, chloro, bromo or iodo, then R⁵ is not 5'-deoxy-ribofuranosyl or 5'-amino-5'-deoxyribofuranosyl;
or a pharmaceutically acceptable salt thereof, that is effective in treating or preventing such disorder. The foregoing method is hereinafter also referred to as "Method A".

The term "halo", as used herein, unless otherwise indicated, includes chloro, fluoro, bromo and iodo.

The term "alkyl", as used herein, unless otherwise indicated, includes saturated monovalent hydrocarbon radicals having straight, branched or cyclic moieties or combinations thereof.

The term "one or more substituents," as used herein, includes from one to the maximum number of substituents possible based on the number of available bonding sites.

The compounds of formula I have chiral centers and therefore exist in different enantiomeric forms. This invention relates to all optical isomers and all stereoisomers of compounds of the formula I, and mixtures thereof.

Formula I above includes compounds identical to those depicted but for the fact that one or more hydrogen, nitrogen or carbon atoms are replaced by radioactive isotopes thereof. Such radiolabelled compounds are useful as research and diagnostic tools in metabolism pharmokinetic studies and in binding assays.

"Ischemic neuronal damage" includes, for example, neuronal damage resulting from CNS surgery, open heart surgery or procedures during which the function of the cardiovascular system is compromised.

"Stress induced immune dysfunctions" include deficiencies in the immune system in both man and animals that are stress induced. For example, such dysfunctions include (but are not limited to) porcine stress syndrome, obsessive-compulsive disorder, bovine shipping fever, equine paroxysmal fibrillation, phobias and dysfunctions induced by confinement stress in livestock animals (e.g., chicken, cows, pigs, sheep, etc.), sheering stress in sheep or human-animal interaction related stress in dogs. (See DeRoth, L, "Stress and Disease in Domestic Animals", pp. 285 et al. in Annals New York Acc. Sci., 1993; Liptrap, R.M., "Stress and Reproduction in Domestic Animals", pp. 275-282 in Annals New York Acc. Sci., 1993; and Dallaire, A., "Stress and Behavior in Domestic Animals", p. 269-273 in Annals New York Acc. Sci., 1993).

This invention also relates to a method of treating or preventing a disorder selected from head trauma, spinal cord trauma, ischemic neuronal damage (e.g., cerebral ischemia such as cerebral hippocampal ischemia), excitotoxic neuronal damage, epilepsy, stroke, stress induced immune dysfunctions, phobias, muscular spasms, Parkinson's disease, Huntington's disease, urinary incontinence, senile dementia of the Alzheimer's type, multiinfarct dementia, amyotrophic lateral sclerosis, chemical dependencies and additions (e.g., dependencies on alcohol, cocaine, heroin, benzodiazepines, or other drugs), and hypoglycemia in a mammal, including a human, comprising administering to such mammal a CRF receptor antagonizing amount of a compound of the formula I, as depicted and defined above, or a pharmaceutically acceptable salt thereof. The foregoing method is hereinafter also referred to as "Method B".

Preferred embodiments of this invention include Methods A and B, as described above, wherein the compound of formula I or the pharmaceutically acceptable salt of such compound that is employed is a compound wherein X is nitrogen and: (a) R¹ is C₁-C₄ alkyl, -(C₂-C₄ alkylene)O(C₁-C₄ alkyl), or C₂-C₄ hydroxyalkyl; (b) R² is C₁-C₅ alkyl, benzyl, phenylethyl, or benzyl substituted with one or two substituents independently selected from chloro, fluoro, methyl, ethyl, methoxy, ethoxy and t-butyl, or by one trifluoromethyl group; (2-thienyl)methyl; (2-thienyl)ethyl; (2-furanyl)methyl; 2-(4-chlorothienyl)methyl; (2-benzofuranyl)methyl; (2-benzothienyl)methyl; (2-thiazolyl)methyl; or (2-benzothiazolyl)methyl; (c) R³ is hydrogen, methyl, ethyl, methoxy, fluoro or chloro; (d) R⁴ is hydrogen, methyl, ethyl, or n-propyl; and (e) R⁵ is phenyl substituted with two or three substituents.

Other preferred embodiments of this invention include Methods A and B, as described above, wherein the compound of formula I or the pharmaceutically acceptable salt of such compound that is employed is a compound wherein X is CR⁶ and: (a) R¹ is C₁-C₄ alkyl, -(C₂-C₄ alkylene)O(C₁-C₄ alkyl), or C₂-C₄ hydroxyalkyl; (b) R² is C₁-C₅ alkyl, benzyl, phenylethyl, or benzyl substituted with one or two substituents independently selected from chloro, fluoro, methyl, ethyl, methoxy, ethoxy and t-butyl, or by one trifluoromethyl group; (2-thienyl)methyl; (2-thienyl)ethyl; (2-furanyl)methyl; 2-(4-chlorothienyl)methyl; (2-benzofuranyl)methyl; (2-benzothienyl)methyl; (2-thiazolyl)methyl; or (2-benzothiazolyl)methyl; (c) R³ is hydrogen, methyl, ethyl, methoxy, fluoro or chloro; (d) R⁴ is hydrogen, methyl, ethyl, or n-propyl; (e) R⁵ is phenyl substituted with two or three substituents; and (f) R⁶ is hydrogen, methyl, ethyl or chloro; with the proviso that R² and R⁶ are not both hydrogen.

Other preferred embodiments of this invention include Methods A and B, as described above, wherein the compound of formula I or the pharmaceutically acceptable salt of such compound that is employed is a compound wherein X is CR⁶ and: (a) A is -NR¹R², -NHCHR¹R², or -OCHR¹R², wherein R¹ is C₁-C₆ alkyl which may optionally be substituted with one of hydroxy, fluoro and C₁-C₂ alkoxy, and may optionally contain one double or triple bond; (b) R² is benzyl or C₁-C₆ alkyl which may optionally contain one double or triple bond, wherein said C₁-C₆ alkyl or the phenyl moiety of said benzyl may optionally be substituted with one fluoro, C₁-C₆ alkyl, or C₁-C₆ alkoxy; (c) R³ is methyl, ethyl, fluoro, chloro or methoxy; (d) R⁴ and R⁶ are independently selected from hydrogen, methyl and ethyl; and (e) R⁵ is phenyl substituted with two or three substituents, said substituents being independently selected from fluoro, chloro, bromo, iodo, C₁-C₄ alkoxy, trifluoromethyl, C₁-C₆ alkyl which may optionally be substituted with one hydroxy group, C₁-C₄ alkoxy and fluoro and which may optionally contain one double or triple bond, -(C₁-C₄ akylene)O(C₁-C₂ alkyl), C₁-C₃ hydroxyalkyl, hydroxy, formyl, -COO(C₁-C₂ alkyl), or -C(O)(C₁-C₄ alkyl).

Other preferred embodiments of this invention include Methods A and B, as described above, wherein the compound of formula I or the pharmaceutically acceptable salt of such compound that is employed is a compound wherein X is nitrogen and: (a) A is -NR¹R², -NHCHR¹R², or -OCHR¹R², wherein R¹ is C₁-C₆ alkyl which may optionally be substituted with one of hydroxy, fluoro and C₁-C₂ alkoxy, and may optionally contain one double or triple bond; (b) R² is benzyl or C₁-C₆ alkyl which may optionally contain one double or triple bond, wherein said C₁-C₆ alkyl or the phenyl moiety of said benzyl may optionally be substituted with one fluoro, C₁-C₆ alkyl, or C₁-C₆ alkoxy; (c) R³ is methyl, ethyl, fluoro, chloro or methoxy; (d) R⁴ and R⁶ are independently selected from hydrogen, methyl and ethyl; and (e) R⁵ is phenyl substituted with two or three substituents, said substituents being independently selected from fluoro, chloro, bromo, iodo, C₁-C₄ alkoxy, trifluoromethyl, C₁-C₆ alkyl which may optionally be substituted with one hydroxy group, C₁-C₄ alkoxy and fluoro and which may optionally contain one double or triple bond, -(C₁-C₄ alkylene)O(C₁-C₂ alkyl), C₁-C₃ hydroxyalkyl, hydroxy, formyl, -COO(C₁-C₂ alkyl), or -C(O)(C₁-C₄ alkyl).

Other embodiments of this invention include Methods A and B, as described above, wherein the compound of formula I or the pharmaceutically acceptable salt of such compound that is employed is a compound wherein X is nitrogen and: (a) A is -NR¹R², -NHCHR¹R², or -OCHR¹R², wherein R¹ is C₁-C₆ alkyl, which may optionally be substituted by one hydroxy, fluoro or C₁-C₂ alkoxy group, and which may optionally contain one double or triple bond, and R² is benzyl or C₁-C₆ alkyl which may optionally contain one double or triple bond, and wherein said C₁-C₆ alkyl or the phenyl moiety of said benzyl may optionally be substituted with fluoro, C₁-C₆ alkyl, or C₁-C₆ alkoxy; or (b) A is -CR¹ R²R¹¹ wherein R¹ is C₁-C₆ alkyl which may optionally be substituted with one C₁-C₆ alkoxy or hydroxy group, R² is benzyl or C₁-C₆ alkyl wherein said C₁-C₆ alkyl or the phenyl moiety of said benzyl may optionally be substituted by one C₁-C₆ alkyl, C₁-C₆ alkoxy, fluoro, chloro or bromo group, and R¹¹ is hydrogen or fluoro.

Other specific embodiments of this invention include Methods A and B, as described above, wherein the compound of formula I or the pharmaceutically acceptable salt of such compound that is employed is a compound wherein X is nitrogen and R² is -(C₁-C₄ alkylene)aryl, wherein said aryl is phenyl, thienyl, benzofuranyl, furanyl, benzothienyl, thiazolyl, pyridyl or benzothiazolyl.

Other specific embodiments of this invention include methods A and B, as described above, wherein the compound of formula I or the pharmaceutically acceptable salt of such compound that is employed is a compound wherein X is nitrogen and R² is a benzyl group that is optionally substituted on the phenyl moiety with one ethyl, t-butyl, methoxy, trifluoromethyl, nitro, fluoro chloro, or methyl group.

Other specific embodiments of this invention include Methods A and B, as described above, wherein the compound of formula I or the pharmaceutically acceptable salt of such compound that is employed is a compound wherein X is nitrogen and R² is attached through a methylene or ethylene bridge to quinolyl, pyrrolyl, pyrrolidinyl, pyridyl, tetrahydropyranyl, cyclopropyl, piperidinyl, or benzyl-piperidinyl.

Other specific embodiments of this invention include Methods A and B, as described above, wherein the compound of formula I or the pharmaceutically acceptable salt of such compound that is employed is a compound wherein X is nitrogen or CR⁶ and R¹ and R² are, independently, C₁-C₆ alkyl which may optionally be substituted with one substitutent selected from hydroxy, methoxy, ethoxy, chloro, fluoro, -OC(O)CH₃, -OC(O)NHCH₃, and -C(O)NH₂.

Other specific embodiments of this invention include Methods A and B, as described above, wherein the compound of formula I or the pharmaceutically acceptable salt of such compound that is employed is a compound wherein X is nitrogen or CR⁶ and R² is C₁-C₆ alkyl which may optionally be substituted with one substituent selected from methoxy and ethoxy.

Other specific embodiments of this invention include Methods A and B, as described above, wherein the compound of formula I or the pharmaceutically acceptable salt of such compound that is employed is a compound wherein X is nitrogen and A is -NR¹R² or -CHR¹R² in which R¹ and R², together with the N or CH to which they are attached, form a 5- or 6-membered ring in which one of the ring carbon atoms may optionally be replaced by an oxygen or sulfur atom, e.g., pyrrolidinyl, pyrrolyl, pyrazolyl, imidazolyl, oxazolyl, thiazolyl, isoxazolyl, thiadiazolyl, oxadiazolyl, pyridyl, pyrazinyl or pyrimidyl.

Other specific embodiments of this invention include Methods A and B, as described above, wherein the compound of formula I or the pharmaceutically acceptable salt of such compound that is employed is a compound wherein X is nitrogen or CR⁶ and A is -NHCHR¹R² or -OCHR¹R² in which -CHR¹R² is a 5- or 6-membered ring in which one of the ring carbon atoms may optionally be replaced by an oxygen or sulfur atom, e.g., tetrahydrofuranyl, tetrahydrothiafuranyl and cyclopentanyl.

Other specific embodiments of this invention include Methods A and B, as described above, wherein the compound of formula I or the pharmaceutically acceptable salt of such compound that is employed is a compound selected from the following:
3-{(4-methyl-benzyl)-[3,6-dimethyl-1-(2,4,6-trimethylphenyl)-1H-pyrazolo[3,4-d]pyrimidin-4-yl]-amino}-propan-1-ol;
diethyl-[6-methyl-3-methylsulfanyl-1-(2,4,6-trichlorophenyl)-1H-pyrazolo[3,4-d]pyrimidin-4-yl]-amine;
2-{butyl-[6-methyl-3-methylsulfanyl-1-(2,4,6-trichlorophenyl)-1H-pyrazolo[3,4-d]pyrimidin-4-yl]-amino}-ethanol;
dibutyl-[6-methyl-3-methylsulfanyl-1-(2,4,6-trichlorophenyl)-1H-pyrazolo[3,4-d]pyrimidin-4-yl}-amine;
butyl-ethyl-[6-methyl-3-methylsulfanyl-1-(2,4,6-trichlorophenyl)-1 H-pyrazolo [3,4-d]pyrimidin-4-yl]-amine;
butyl-ethyl-[6-methyl-3-methylsulfonyl-1-(2,4,6-trichlorophenyl)-1H-pyrazolo[3,4-d]pyrimidin-4-yl]-amine;
butyl-cyclopropylmethyl-[6-methyl-3-methylsulfanyl-1-(2,4,6-trichlorophenyl)-1H-pyrazolo [3,4d]pyrimidin-4-yl]-amine;
di-1-propyl-[6-methyl-3-methylsulfanyl-1-(2,4,6-trichlorophenyl)-1H-pyrazolo[3,4-d]pyrimidin-4-yl]-amine;
diallyl-[6-methyl-3-methylsulfanyl-1-(2,4,6-trichlorophenyl)-1H-pyrazolo[3,4-d]pyrimidin-4-yl]-amine;
butyl-ethyl-[6-chloro-3-methylsulfanyl-1-(2,4,6-trichlorophenyl)-1H-pyrazolo[3,4-d]pyrimidin-4-yl]-amine;
butyl-ethyl-[6-methoxy-3-methylsulfanyl-1-(2,4,6-trichlorophenyl)-1H-pyrazolo[3,4-d]pyrimidin-4-yl]-amine;
propyl-ethyl-[3,6-dimethyl-1-(2,4,6-trimethylphenyl)-1H-pyrazolo[3,4,6-d]pyrimidin-4-yl]-amine;
4-(1-ethyl-propyl)-6-methyl-3-methylsulfanyl-1-(2,4,6-trimethylphenyl)-1H-pyrazolo[3,4-d]pyrimidine;
2-[3,6-dimethyl-1-(2,4,6-trimethylphenyl)-1H-pyrazolo[3,4-d]pyrimidin-4-ylamine]-butan-1-ol;
[3,6-dimethyl-1-(2,4,6-trimethylphenyl)-1H-pyrazolo-[3,4-d]pyrimidin-4-yl]-(1-methylpropyl)amine;
4-(1-methoxymethylpropoxy)-3,6-dimethyl-1-(2,4,6-trimethylphenyl)-1H-pyrazolo[3,4-d]pyrimidine;
n-butyl-ethyl-[2,6-dimethyl-7-(2,4,6-trimethylphenyl)-7H-pyrrolo[2,3-d]pyrimidin-4-yl]amine;
di-n-propyl-[2,5-dimethyl-7-(2,4,6-trimethylphenyl)-7H-pyrrolo[2,3-d]pyrimidin-4-yl]amine;
ethyl-n-propyl-[2,5-dimethyl-7-(2,4,6-timethylphenyl)-7H-pyrrolo[2,3-d]pyrimidin-4-yl]amine;
diethyl-2,5-dimethyl-7-(2,4,6-trimethylphenyl)-7H-pyrrolo[2,3-d]pyrimidin-4-yl]amine;
n-butyl-ethyl-[2,5,6-trimethyl-7-(2,4,6-trimethylphenyl)-7H-pyrrolo[2,3-d]pyrimidin-4-yl]amine;
2-{N-n-buty-N-[2,5-dimethyl-7-(2,4,6-trimethylphenyl)-7H-pyrrolo[2,3-d]pyrimidin-4-yl]amino}-ethanol;
4-(1-ethyl-propyl)-2,5,6-trimethyl-7-(2,4,6-trimethylphenyl)-7H-pyrrolo[2-3-d]pyrimidine;
n-butyl-ethyl-[2,5-dimethyl-7-(2,4-dimethylphenyl)-7H-pyrrolo[2,3-d]pyrimidin-4-yl]amine;
2,5-dimethyl-7-(2,4,6-trimethylphenyl)-7H-pyrrolo[2,3-d]pyrimidyl-4-yl]-(1-ethylpropyl)amine;
2-[7-(4-bromo-2,6-dimethylphenyl)-2,5-dimethyl-7H-pyrrolo [2,3-d]pyrimidin-4-ylamino]-butan-1-ol;
2-(S)-[7-(4-bromo-2,6-dimethylphenyl)-2,5-dimethyl-7H-pyrrolo[2,3-d]pyrimidin-4-ylamino]-butan-1-ol;
4-(1-ethyl-propoxy)-2,5,6-trimethyl-7-(2,4,6-trimethylphenyl)-7H-pyrrolo[2,3-d]pyrimidine;
4-(1-methoxymethyl-propoxy)-2,5,6-trimethyl-7-(2,4,6-trimethylphenyl)-7H-pyrrolo[2,3-d]pyrimidine;
4-(1-ethyl-butyl)-2,5,6-trimethyl-7-(2,4,6-trimethylphenyl)-7H-pyrrolo-[2,3-d]pyrimidine;
[7-(4-bromo-2,6-dimethyl-phenyl)-2,5-dimethyl-7H-pyrrolo[2,3-d]pyrimidin-4-yl]-(1-methoxymethyl-propyl)-amine;
2-[7-(2-bromo-4,6-dimethyl-phenyl)-2,5-dimethyl-7H-pyrrolo[2,3-d]pyrimidin-4-ylamino]-butan-1-ol;
2-[7-(4-ethyl-2,6-dimethyl-phenyl)-2,5-dimethyl-7H-pyrrolo[2,3-d]pyrimidin-4-ylamino]-butan-1-ol;
2-[7-(2-ethyl-4,6-dimethylphenyl)-2,5-dimethyl-7H-pyrrolo[2,3-d]pyrimidin-4-ylamino]-butan-1-ol; and
2-[7-(2-fluoromethyl-4,6-dimethylphenyl)-2,5-dimethyl-7H-pyrrolo[2,3-d]pyrimidin-4-ylamino]-butan-1-ol.

Whenever reference is made to alkyl, this includes both straight and branched chain alkyl.

Whenever reference is made herein to 3-to 8-membered cycloalkyl or 9- to 12-membered bicycloalkyl containing one to three of O, S or N-Z, it is understood that the oxygen and sulfur ring atoms are not adjacent to each other. The three membered cycloalkyl has just one O, S or N-Z. An example of a six-membered cycloalkyl having O and N is morpholinyl.

Whenever R² or R⁵ is a heterocyclic group, the attachment of the group is through a carbon atom.

Whenever reference is made herein to C₁-C₄ alkyl or C₁-C₆ alkyl which "may contain one or two double or triple bonds" in the definitions of R¹, R² and R³, it is understood that at least two carbons are present in the alkyl for one double or triple bond, and at least four carbons for two double and triple bonds.

Whenever an alkoxy group, e.g., in the definitions of R¹ and R², may have a double or triple bond, it is understood that such double or triple bond is not directly attached to the oxygen.

Formula I above is intended to include all stereoisomers (e.g., all geometric and optical isomers) as well as all racemates of all individual compounds within the depicted genus.

### Detailed Description of the Invention

Compounds of the formula I wherein X is nitrogen and the pharmaceutically acceptable acid addition of such compounds, as well as methods for preparing such compounds and salts, are referred to in World Patent Application PCT/US 93/11333, which was filed on November 26, 1993.

Compounds of the formula I wherein X is CR⁶ and the pharmaceutically acceptable acid addition of such compounds, as well as methods of preparing such compounds and salts, are referred to in World Patent Application PCT/US 93/10715, which was filed on 12 November 1993.

For use in carrying out the methods of this invention, compounds of the formula I and their pharmaceutically acceptable salts may be administered orally, topically or parenterally. They may be administered alone or in combination with pharmaceutically acceptable carriers, in either single or multiple, e.g. up to three, doses. Suitable pharmaceutical carriers include inert solid diluents or fillers, sterile aqueous solution and various organic solvents. The pharmaceutical compositions formed by combining the novel compounds of formula I and the pharmaceutically acceptable carriers are then readily administered in a variety of dosage forms such as tablets, powders, lozenges, syrups, injectable solutions and the like. These pharmaceutical compositions can, if desired, contain additional ingredients such as flavorings, binders, excipients and the like. Thus, for purposes of oral administration, tablets containing various excipients such as sodium citrate, calcium carbonate and calcium phosphate may be employed along with various disintegrants such as starch, alginic acid and certain complex silicates, together with binding agents such as polyvinylpyrrolidone, sucrose, gelatin and acacia. Additionally, lubricating agents such as magnesium stearate, sodium lauryl sulfate and talc are often useful for tabletting purposes. Solid compositions of a similar type may also be employed as fillers in soft and hard filled gelatin capsules. Preferred materials for this include lactose or milk sugar and high molecular weight polyethylene glycols. Oral compositions may also be administered in the form of a gel. When aqueous suspensions or elixirs are desired for oral administration, the essential active ingredient therein may be combined with various sweetening or flavoring agents, coloring matter or dyes and, if desired, emulsifying or suspending agents, together with diluents such as water, ethanol, propylene glycol, glycerin and combinations thereof.

For parenteral administration, solutions of the novel compound of formula I in sesame or peanut oil, aqueous propylene glycol, or in sterile aqueous solution may be employed. Such aqueous solutions should be suitably buffered if necessary and the liquid diluent first rendered isotonic with sufficient saline or glucose. These particular aqueous solutions are especially suitable for intravenous, intramuscular, subcutaneous and intraperitoneal administration. The sterile aqueous media employed are all readily available by standard techniques known to those skilled in the art.

Additionally, it is possible to administer the compounds employed in the method of the present invention topically when treating inflammatory conditions of the skin and this may be done by way of creams, jellies, gels, pastes and ointments in accordance with standard pharmaceutical practice.

The compounds of formula I and the pharmaceutically acceptable salts thereof will generally be administered from one to three times per day (i.e., from one to three doses per day), with each dose containing from about 0.1 to about 100 mg/kg body weight, although variations will necessarily occur depending upon the weight and condition of the subject being treated, the nature and severity of the disorder for which the subject is being treated and the particular route of administration chosen. However, a dosage level that is in the range of about 1.0 mg to about 50 mg per kg of body weight per individual dose will most desirably be employed. Variations may nevertheless occur depending upon the species of mammal being treated and the individual subject's response to said medicament, as well as on the type of pharmaceutical formulation chosen and the time period and interval at which such administration is carried out.

The methods for testing the compounds for formula I for their CRF antagonist activity are according to the procedures of Endocrinology, 116, 1653-1659 (1985) and Peptides, 10, 179-188 (1985), which determine the binding affinity of a test compound for a CRF receptor. The binding affinities for the compounds of formula I, expressed as IC₅₀ values, generally range from about 0.2 nanomolar to about 10 micromolar.

## Claims

1. Use of a compound of the formula 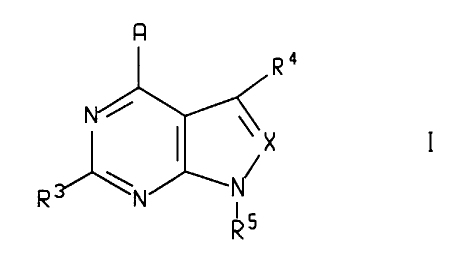 wherein
X is nitrogen or -CR⁶;
A is -NR¹R², -CR¹R²R¹¹, -C(=CR²R¹²)R¹, -NHCR¹R²R¹¹, -OCR¹R²R¹¹, -SCR¹R²R¹¹, -NHNR¹R², -CR²R¹¹NHR¹, -CR²R¹¹OR¹, -CR²R¹¹SR¹, or -C(O)R²;
R¹ is hydrogen, or C₁-C₆ alkyl which may optionally be substituted with from one to two substituents independently selected from the group consisting of hydroxy, fluoro, chloro, bromo, iodo, C₁-C₈ alkoxy, -O-CO-(C₁-C₆ alkyl), -O-CONH(C₁-C₄ alkyl), -O-CO-N(C₁-C₄ alkyl)(C₁-C₂ alkyl), amino, -NH(C₁-C₄ alkyl), -N(C₁-C₂ alkyl)(C₁-C₄ alkyl), -S(C₁-C₆ alkyl), -N(C₁-C₄ alkyl)CO(C₁-C₄ alkyl), -NHCO(C₁-C₄ alkyl), -COOH, -CO-O(C₁-C₄ alkyl), -CONH(C₁-C₄ alkyl), -CON(C₁-C₄ alkyl)(C₁-C₂ alkyl), -SH, -CN, -NO₂, -SO(C₁-C₄ alkyl), -SO₂(C₁-C₄ alkyl), -SO₂NH(C₁-C₄ alkyl), -SO₂N(C₁-C₄ alkyl)(C₁-C₂ alkyl), and wherein each of the foregoing C₁-C₆ alkyl moieties in the definition of R¹ may contain one or two double or triple bonds;
R² is C₁-C₁₂ alkyl, aryl or -(C₁-C₁₀ alkylene)aryl wherein said aryl is phenyl, naphthyl, thienyl, benzothienyl, pyridyl, quinolyl, pyrazinyl, pyrimidyl, imidazolyl, furanyl, benzofuranyl, benzothiazolyl, isothiazolyl, benzisothiazolyl, thiazolyl, isoxazolyl, benzisoxazolyl, benzimidazolyl, triazolyl, pyrazolyl, pyrrolyl, indolyl, pyrrolopyridyl, oxazolyl, or benzoxazolyl; 3- to 8-membered cycloalkyl or -(C₁-C₆ alkylene) cycloalkyl, wherein one or two of the carbon atoms of any of said cycloalkyl moieties may optionally be replaced, independently, by O, S or N-Z wherein Z is hydrogen, C₁-C₄ alkyl or C₁-C₄ alkanoyl, and wherein R² may optionally be substituted with from one to three substituents independently selected from chloro, fluoro and C₁-C₄ alkyl, or by one substituent selected from hydroxy, bromo, iodo, C₁-C₆ alkoxy, 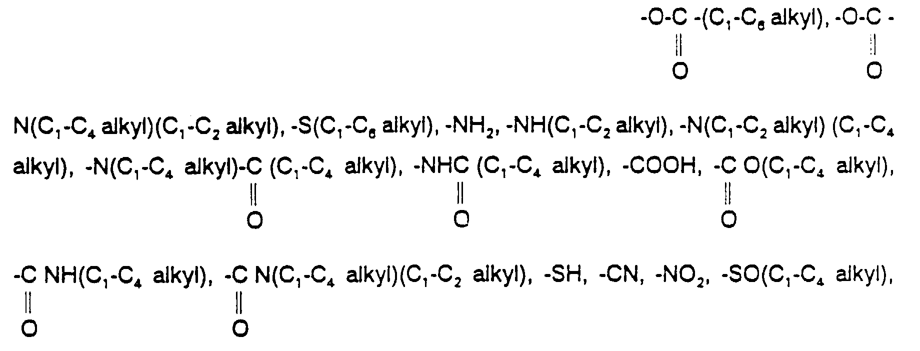 -SO₂(C₁-C₄ alkyl), -SO₂NH(C₁-C₄ alkyl), and -SO₂N(S₁-C₄ alkyl)(C₁-C₂ alkyl), and wherein each of the foregoing C₁-C₁₂ alkyl and C₁-C₁₀ alkylene moieties in the definition may optionally contain one to three double or triple bonds; or
R¹ and R², taken together with the atom to which they are attached, may form a saturated 3- to 8-membered ring which, if it is a 5- to 8-membered ring, may optionally contain one or two double bonds, and wherein one or two of the carbon atoms of said 5- to 8- membered ring may optionally be replaced, independently, by O, S or N-Z wherein Z is hydrogen, C₁-C₄ alkyl, C₁-C₄ alkanoyl or benzyl;
R³ is hydrogen, C₁-C₆ alkyl, fluoro, chloro, bromo, iodo, hydroxy, amino, -O(C₁-C₆ alkyl), -NH(C₁-C₆ alkyl), -N(C₁-C₄ alkyl)(C₁-C₂ alkyl), -SH, -S(C₁-C₄ alkyl), -SO(C₁-C₄ alkyl), or -SO₂(C₁-C₄ alkyl), wherein each of the foregoing C₁-C₄ alkyl and C₁-C₆ alkyl moieties in the definition of R³ may contain one double or triple bond and may optionally be substituted with from 1 to 3 substituents independently selected from the group consisting of hydroxy, C₁-C₃ alkoxy, fluoro, chloro and C₁-C₃ thioalkyl;
R⁴ is hydrogen, C₁-C₆ alkyl, fluoro, chloro, bromo, iodo, C₁-C₆ alkoxy, formyl, -NH(C₁-C₆ alkyl), -N(C₁-C₆ alkyl)(C₁-C₂ alkyl), -SOₙ(C₁-C₆ alkyl), wherein n is 0, or 2, cyano, hydroxy, carboxy, or amido, wherein each of the foregoing C₁-C₆ alkyl moieties in the definition of R⁴ may optionally be substituted with one substituent selected from hydroxy, trifluoromethyl, amino, carboxy, amido, 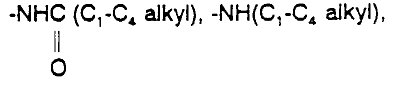 -N(C₁-C₄ alkyl)(C₁-C₂ alkyl), 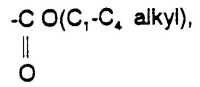 C₁-C₃ alkoxy, C₁-C₃ thioalkyl, fluoro, bromo, chloro, iodo, cyano and nitro;
R⁵ is phenyl, naphthyl, thienyl, benzothienyl, pyridyl, quinolyl, pyrazinyl, pyrimidyl, imidazolyl, furanyl, benzofuranyl, benzothiazolyl, isothiazolyl, benzoisothiazolyl, thiazolyl, isoxazolyl, benzisoxazolyl, benzimidazolyl, triazolyl, pyrazolyl, pyrrolyl, indolyl, pyrrolopyridyl, benzoxazolyl, oxazolyl, pyrrolidinyl, thiazolidinyl, morpholinyl, piperidinyl, piperazinyl, tetrazolyl, or a 3- to 8-membered cycloalkyl or 9- to 12-membered bicycloalkyl ring, wherein one or two of the carbon atoms in said ring may optionally be replaced, independently, by O, S or N-Z wherein Z is hydrogen, C₁-C₄ alkyl, C₁-C₄ alkanoyl, phenyl or benzyl, and wherein each of the above R⁵ groups may optionally be substituted with one or more substituents, preferably with two or three substituents, independently selected from fluoro, chloro, bromo, formyl, C₁-C₆ alkyl, C₁-C₆ alkoxy and trifluoromethyl, or with one substituent selected from hydroxy, iodo, cyano, nitro, amino, -NH(C₁-C₄ alkyl), -N(C₁-C₄ alkyl)(C₁-C₂ alkyl), -COO(C₁-C₄ alkyl), -CO(C₁-C₄ alkyl), -SO₂NH(C₁-C₄ alkyl), -SO₂N(C₁-C₄ alkyl)(C₁-C₂ alkyl), -SO₂NH₂, -NHSO₂(C₁-C₄ alkyl), -S(C₁-C₆ alkyl), -SO₂(C₁-C₆ alkyl), and wherein each of the foregoing C₁-C₄ alkyl and C₁-C₆ alkyl moieties in the definition of R⁵ may optionally be substituted with from one to two substituents independently selected from fluoro, chloro, hydroxy, C₁-C₄ alkoxy, amino, methylamino, dimethylamino and acetyl, and wherein each of the foregoing C₁-C₄ alkyl and C₁-C₆ alkyl moieties in the definition of R⁶ may optionally contain one double or triple bond; with the proviso that R⁶ is not unsubstituted phenyl;
R⁶ is hydrogen, C₁-C₆ alkyl, fluoro, chloro, bromo, iodo, C₁-C₆ alkoxy, formyl, amino, -NH(C₁-C₆ alkyl), N(C₁-C₆ alkyl)(C₁-C₂ alkyl), -SOₙ(C₁-C₆ alkyl), wherein n is 0, 1 or 2, cyano, carboxy, or amido, and wherein each of the foregoing (C₁-C₆)alkyl moieties in the definition of R⁶ may be optionally substituted with one substituent selected from hydroxy, trifluoromethyl, amino, carboxy, amido, 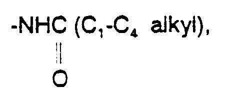 -NH(C₁-C₄ alkyl), -N(C₁-C₄ alkyl)(C₁-C₂ alkyl), 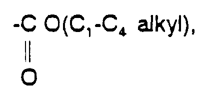 C₁-C₃ alkoxy, C₁-C₃ thioalkyl, fluoro, bromo, chloro, iodo, cyano and nitro;
R¹¹ is hydrogen, hydroxy, fluoro, chloro, -COO(C₁-C₂ alkyl), cyano, or -CO(C₁-C₂ alkyl); and
R¹² is hydrogen or C₁-C₄ alkyl;
with the proviso that: (1) when X is -CR⁶, A is not straight chain alkyl, (2) when X is -CR⁶ and R⁵ is unsubstituted cycloalkyl and R³ and R⁴ are both hydrogen and R⁶ is hydrogen or methyl, then A is not -NHR² wherein R² is benzyl or thienylmethyl, and (3) when X is -CR⁶ and R⁵ is p-bromophenyl and R³, R⁴ and R⁶ are methyl, then A is not methylamino or hydroxyethylamino;
and when X is nitrogen, with the further proviso that:
(a) A is not straight chain C₁-C₁₂ alkyl;
(b) R⁵ is not a sugar group;
(c) when R³ and R⁴ are hydrogen and R⁵ is chlorophenyl, then A is not -NH-CH(CH₃)-(CH₂)₃-N(C₂H₅)₂;
(d) when R³ and R⁴ are hydrogen and A is -NR¹ R² wherein R¹ is C₃-C₇ cycloalkyl, and R² is C₂-C₆ alkenyl, phenyl-(C₁-C₆ alkylene) or hetero-(C₁-C₆ alkylene) wherein the hetero radical is furyl, thienyl or pyridinyl, and wherein said phenyl may be substituted by fluoro, chloro, bromo or iodo, then R⁵ is not tetrahydrofuranyl or tetrahydropyranyl;
(e) when R³ is methoxy, methylthio, or methylsulfonyl, R⁴ is hydrogen, and R⁵ is tetrahydrofuranyl or tetrahydropyranyl, then A is not -NH(C₁-C₂-alkyl), morpholinyl, hydrazino, or -NHC₂H₄C₆H₅, the phenyl of which may be substituted by one methyl or two methoxy groups;
(f) when R³ is hydrogen, C₁-C₆ alkyl, chloro, bromo, -SH, or -S-(C₁-C₄ alkyl), R⁴ is hydrogen and R⁵ is C₃-C₆ cycloalkyl, then A is not hydrazino, -NH(C₁-C₂ alkyl) or -N(C₁-C₆ alkyl) (C₁-C₁₂ alkyl);
(g) when R³ and R⁴ are hydrogen and A is -NH(CH₂)ₘ COOH wherein m is 1-12, then R⁵ is not phenyl substituted by one fluoro, chloro, bromo or iodo group;
(h) when R³ is hydrogen, hydroxy, methylthio, chloro or -NHbenzyl, R⁴ is hydrogen, and R⁵ is chlorophenyl or bromophenyl, then A is not -NH(C₁-C₁₂ alkyl), -NHallyl, or -N(C₁-C₆ alkyl) (C₁-C₁₂ alkyl), wherein said C₁-C₁₂ alkyl may be substituted by -NC₂H₅, or -NH benzyl which may be substituted by one or two bromo, chloro, fluoro, -NC₂H₅ phenyl or morpholinopropyl groups;
(i) when R³ and R⁴ are hydrogen and R⁵ is nitrophenyl, then A is not -NHR² wherein R² is phenyl, benzyl or C₁-C₁₂ alkyl which may be substituted by one or two hydroxy groups;
(j) when R³ is chloro or -O(C₁-C₆ alkyl), R⁴ is hydrogen, and A is -NR¹R² wherein R¹ and R² are independently hydrogen or C₁-C₆ alkyl, then R⁵ is not chlorophenyl; and
(k) when R³ is hydrogen, A is benzyl or phenethyl, and R⁴ is fluoro, chloro, bromo or iodo, then R⁵ is not 5'-deoxy-ribofuranosyl or 5'-amino-5'-deoxyribofuranosyl;
or a pharmaceutically acceptable salt thereof;
in the manufacture of a medicament for preventing or inhibiting a disorder selected from head traumas, spinal cord trauma, ischemic neuronal damage, excitotoxic neuronal damage, epilepsy, stroke, stress induced immune dysfunctions, phobias, muscular spasms, Parkinson's disease, Huntington's disease, urinary incontinence, senile dementia of the Alzheimer's type, multiinfarct dementia, amyotrophic lateral sclerosis, chemical dependencies and addictions and hypoglycemia.

2. The use as claimed in claim 1, wherein a dose of the medicament contains a quantitiy of a compound of formula I, or a pharmaceutically acceptable salt thereof, capable of antagonizing the effects of CRF in a mammal.

3. The use according to claim 1 or claim 2, wherein the compound of formula I or the pharmaceutically acceptable salt of such compound that is employed is a compound wherein X is nitrogen and: (a) R¹ is C₁-C₄ alkyl, -(C₂-C₄ akyyene)O(C₁-C₄ alkyl), or C₂-C₄ hydroxyalkyl; (b) R² is C₁-C₅ alkyl, benzyl, phenylethyl, or benzyl substituted with one or two substituents independently selected from chloro, fluoro, methyl, ethyl, methoxy, ethoxy and t-butyl, or with one trifluoromethyl group; (2-thienyl)methyl; (2-thienyl)ethyl; (2-furanyl)methyl; 2-(4-chlorothienyl)methyl; (2-benzofuranyl)methyl; (2-benzothienyl)methyl; (2-thiazolyl) methyl; or (2-benzothiazolyl)methyl; (c) R³ is hydrogen, methyl, ethyl, methoxy, fluoro or chloro; (d) R⁴ is hydrogen, methyl, ethyl, or n-propyl; and (e) R⁵ is phenyl substituted by two or three substituents.

4. The use according to claim 1 or claim 2, wherein the compound of formula I or the pharmaceutically acceptable salt of such compound that is employed is a compound wherein X is -CR⁶ and: (a) R¹ is C₁-C₄ alkyl, -(C₂-C₄ alkylene)O(C₁-C₄ alkyl), or C₂-C₄ hydroxyalkyl; (b) R² is C₁-C₅ alkyl, benzyl, phenylethyl, or benzyl substituted with one or two substituents independently selected from chloro, fluoro, methyl, ethyl, methoxy, ethoxy and t-butyl, or with one trifluoromethyl group; (2-thienyl)methyl; (2-thienyl)ethyl; (2-furanyl)methyl; 2-(4-chlorothienyl)methyl; (2-benzofuranyl)methyl; (2-benzothienyl)methyl; (2-thiazolyl) methyl; or (2-benzothiazolyl)methyl; (c) R³ is hydrogen, methyl, ethyl, methoxy, fluoro or chloro; (d) R⁴ is hydrogen, methyl, ethyl, or n-propyl; (e) R⁵ is phenyl substituted by two or three substituents; and (f) R⁶ is hydrogen, methyl, ethyl or chloro; with the proviso that R² and R⁶ are not both hydrogen.

5. The use according to claim 1 or claim 2, wherein the compound of formula I or the pharmaceutically acceptable salt of such compound that is employed is a compound wherein X is nitrogen and: (a) A is -NR¹R², -NHCHR¹R², or -OCHR¹R², wherein R¹ is C₁-C₆ alkyl, which may optionally be substituted by one hydroxy, fluoro or C₁-C₂ alkoxy group, and which may optionally contain one double or triple bond, and R² is benzyl or C₁-C₆ alkyl which may optionally contain one double or triple bond, wherein said C₁-C₆ alkyl or the phenyl moiety of said benzyl may optionally be substituted with one fluoro, C₁-C₆ alkyl, or C₁-C₆ alkoxy group; or (b) A is -CR¹R²R¹¹ wherein R¹ is C₁-C₆ alkyl which may optionally be substituted with one C₁-C₆ alkoxy or hydroxy group, R² is benzyl or C₁-C₆ alkyl wherein said C₁-C₆ alkyl or the phenyl in said benzyl may optionally be substituted by one C₁-C₆ alkyl, C₁-C₆ alkoxy, fluoro, chloro or bromo group, and R¹¹ is hydrogen or fluoro.

6. The use according to claim 1 or claim 2, wherein the compound of formula I, or the pharmaceutically acceptable salt of such compound that is employed is a compound wherein X is nitrogen and R² is -(C₁-C₄ alkylene)aryl wherein said aryl is phenyl, thienyl, benzofuranyl, furanyl, benzothienyl, thiazolyl, pyridyl or benzothiazolyl.

7. The use according to claim 1 or claim 2, wherein the compound of formula I, or the pharmaceutically acceptable salt of such compound that is employed is a compound wherein X is nitrogen and R² is benzyl optionally substituted on the phenyl moiety with one ethyl, t-butyl, methoxy, trifluoromethyl, nitro, fluoro, chloro, or methyl group.

8. The use according to claim 1 or claim 2, wherein the compound of formula I, or the pharmaceutically acceptable salt of such compound that is employed is a compound wherein X is nitrogen and R² is attached through a methylene or ethylene bridge to quinolyl, pyrrolyl, pyrrolidinyl, pyridyl, tetrahydropyranyl, cyclopropyl, piperidinyl, or benzyl-piperidinyl.

9. The use according to claim 1 or claim 2, wherein the compound of formula I, or the pharmaceutically acceptable salt of such compound that is employed is a compound wherein X is nitrogen or -CR⁶ and R¹ and R² are, independently, C₁-C₆ alkyl which may optionally be substituted with one hydroxy, methoxy, ethoxy, chloro, fluoro, -OC(O)CH₃, -OC(O)NHCH₃, or -C(O)NH₂ group.

10. The use according to claim 1 or claim 2, wherein the compound of formula I, or the pharmaceutically acceptable salt of such compound that is employed is a compound wherein X is nitrogen or -CR⁶ and R² is C₁-C₆ alkyl substituted with one substituent selected from methoxy and ethoxy.

11. The use according to claim 1 or claim 2, wherein the compound of formula I, or the pharmaceutically acceptable salt of such compound that is employed is a compound wherein X is nitrogen and A is -NR¹R² or -CHR¹R² in which R¹ and R², together with the N or CH to which they are attached, form a 5- or 6-membered ring in which one of the ring carbons may optionally be replaced by an oxygen or sulfur atom.

12. The use according to claim 1 or claim 2, wherein the compound of formula I, or the pharmaceutically acceptable salt of such compound that is employed is a compound wherein X is nitrogen or CR⁶ and A is -NHCHR¹R² or -OCHR¹R² in which CHR¹R² is a 5- or 6-membered ring in which one of the ring carbons may optionally be replaced by an oxygen or sulfur atom.

13. The use according to claim 1 or claim 2, wherein the compound of formula I or the pharmaceutically acceptable salt of such compound that is employed is a compound wherein X is nitrogen and: (a) A is -NR¹R², -NHCHR¹R², or -OCHR¹R², wherein R¹ is C₁-C₆ alkyl, which may optionally be substituted with one hydroxy, fluoro or C₁-C₂ alkoxy group and may optionally contain one double or triple bond; (b) R² is benzyl or C₁-C₆ alkyl which may optionally contain one double or triple bond, wherein said C₁-C₆ alkyl or the phenyl in said benzyl may optionally be substituted with fluoro, C₁-C₆ alkyl, or C₁-C₆ alkoxy; (c) R³ is methyl, ethyl, fluoro, chloro or methoxy; (c) R⁴ and R⁶ are independently selected from hydrogen, methyl and ethyl; and (d) R⁵ is phenyl substituted by two or three substituents, said substituents being independently selected from fluoro, chloro, bromo, iodo, C₁-C₄ alkoxy, trifluoromethyl, C₁-C₆ alkyl which may optionally be substituted with one hydroxy, C₁-C₄ alkoxy or fluoro group and which may optionally contain one double or triple bond, -(C₁-C₄ alkylene)O(C₁-C₂ alkyl), C₁-C₃ hydroxyalkyl, hydroxy, formyl, -COO(C₁-C₂ alkyl) and -C(O)(C₁-C₄ alkyl).

14. The use according to claim 1 or claim 2, wherein the compound of formula I or the pharmaceutically acceptable salt of such compound that is employed is a compound wherein X is CR⁶ and: (a) A is -NR¹R², -NHCHR¹R², or -OCHR¹R², wherein R¹ is C₁-C₆ alkyl, which may optionally be substituted with one hydroxy, fluoro or C₁-C₂ alkoxy group and may optionally contain one double or triple bond; (b) R² is benzyl or C₁-C₆ alkyl which may optionally contain one double or triple bond, wherein said C₁ -C₆ alkyl or the phenyl in said benzyl may optionally be substituted with fluoro, C₁-C₆ alkyl, or C₁-C₆ alkoxy; (c) R³ is methyl, ethyl, fluoro, chloro or methoxy; (c) R⁴ and R⁶ are independently selected from hydrogen, methyl and ethyl; and (d) R⁵ is phenyl substituted by two or three substituents, said substituents being independently selected from fluoro, chloro, bromo, iodo, C₁-C₄ alkoxy, trifluoromethyl, C₁-C₆ alkyl which may optionally be substituted with one hydroxy, C₁-C₄ alkoxy or fluoro group and which may optionally contain one double or triple bond, -(C₁-C₄ alkylene)O(C₁-C₂ alkyl), C₁-C₃ hydroxyalkyl, hydroxy, formyl, -COO(C₁-C₂ alkyl), and -C(O)(C₁-C₄ alkyl).

15. The use according to claim 1 or claim 2, wherein the compound of formula I, or the pharmaceutically acceptable salt of such compound that is employed is a compound selected from the following:
3-{(4-methyl-benzyl)-[3,6-dimethyl-1-(2,4,6-trimethylphenyl)-1H-pyrazolo[3,4-d]pyrimidin-4-yl]-amino)-propan-1-ol;
diethyl-[6-methyl-3-methylsulfanyl-1-(2,4,6-trichlorophenyl)-1H-pyrazolo[3,4-d]pyrimidin-4-yl]-amine;
2-{butyl-[6-methyl-3-methylsulfanyl-1-(2,4,6-trichlorophenyl)-1H-pyrazolo[3,4-d]pyrimidin-4-yl]amino}-ethanol;
dibutyl-[6-methyl-3-methylsulfanyl-1-(2,4,6-trichlorophenyl)-1H-pyrazolo[3,4-d]pyrimidin-4-yl}-amine;
butyl-ethyl-[6-methyl-3-methylsulfanyl-1-(2,4,6-trichlorophenyl)-1H-pyrazolo[3,4-d]pyrimidin-4-yl]-amine;
butyl-ethyl-[6-methyl-3-methylsulfonyl-1-(2,4,6-trichlorophenyl)-1H-pyrazolo[3,4-d]pyrimidin-4-yl]-amine;
butyl-cyclopropylmethyl-[6-methyl-3-methylsulfanyl-1-(2,4,6-trichlorophenyl)-1H-pyrazolo[3,4-d]pyrimidin-4-yl]-amine;
di-1-propyl-[6-methyl-3-methylsulfanyl-1-(2,4,6-trichlorophenyl)-1H-pyrazolo[3,4-d]pyrimidin-4-yl]-amine;
diallyl-[6-methyl-3-methylsulfanyl-1-(2,4,6-trichlorophenyl)-1H-pyrazolo[3,4-d]pyrimidin-4-yl]-amine;
butyl-ethyl-[6-chloro-3-methylsulfanyl-1-(2,4,6-trichlorophenyl)-1H-pyrazolo[3,4-d]pyrimidin-4-yl]-amine;
butyl-ethyl-[6-methoxy-3-methylsulfanyl-1-(2,4,6-trichlorophenyl)-1H-pyrazolo[3,4-d]pyrimidin-4-yl]-amine;
propyl-ethyl-[3,6-dimethyl-1-(2,4,6-trimethylphenyl)-1H-pyrazolo[3,4-d]pyrimidin-4-yl]-amine;
4-(1-ethyl-propyl)-6-methyl-3-methylsulfanyl-1-(2,4,6-trimethylphenyl)-1H-pyrazolo[3,4-d]pyrimidine;
2-[3,6-dimethyl-1-(2,4,6-trimethylphenyl)-1 H-pyrazolo[3,4-d]pyrimidin-4-ylamine]-butan-1-ol;
[3,6-dimethyl-1-(2,4,6-trimethylphenyl)-1H-pyrazolo-[3,4-d]pyrimidin-4-yl]-(1-methylpropyl)amine;
4-(1-methoxymethylpropoxy)-3,6-dimethyl-1-(2,4,6-trimethylphenyl)-1H-pyrazolo[3,4-d]pyrimidine;
n-butyl-ethyl-[2,5-dimethyl-7-(2,4,6-trimethylphenyl)-7H-pyrrolo[2,3-d]pyrimidin-4-yl]amine;
di-n-propyl-[2,5-dimethyl-7-(2,4,6-trimethylphenyl)-7H-pyrrolo[2,3-d]pyrimidin-4-yl]amine;
ethyl-n-propyl-[2,5-dimethyl-7-(2,4,6-trimetylphenyl)-7H-pyrrolo[2,3-d]pyrimidin-4-yl]amine;
diethyl-2,5-dimethyl-7-(2,4,6-trimethylphenyl)-7H-pyrrolo[2,3-d]pyrimidin-4-yl]amine;
n-butyl-ethyl-[2,5,6-trimethyl-7-(2,4,6-trimethylphenyl)-7H-pyrrolo[2,3-d]pyrimidin-4-yl]amine;
2-{N-n-butyl-N-[2,5-dimethyl-7-(2,4,6-trimethylphenyl)-7H-pyrrolo[2,3-d]pyrimidin-4-yl]amino}-ethanol;
4-(1-ethyl-propyl)-2,5,6-trimethyl-7-(2,4,6-trimethylphenyl)-7H-pyrrolo [2,3-d]pyrimidine;
n-butyl-ethyl-[2,5-dimethyl-7-(2,4-dimethylphenyl)-7H-pyrrolo[2,3-d]pyrimidin-4-yl]amine;
2,5-dimethyl-7-(2,4,6-trimethylphenyl)-7H-pyrrolo[2,3-d]pyrimidyl-4-yl]-(1-ethylpropyl)amine;
2-[7-(4-bromo-2,6-dimethylphenyl)-2,5-dimethyl-7H-pyrrolo[2,3-d]pyrimidin-4-ylamino]-butan-1-ol;
2-(S)-[7-(4-bromo-2,6-dimethylphenyl)-2,5-dimethyl-7H-pyrrolo[2,3-d]pyrimidin-4-ylamino]-butan-1-ol;
4-(1-ethyl-propoxy)-2,5,6-trimethyl-7-(2,4,6-trimethylphenyl)-7H-pyrrolo[2,3-d]pyrimidine;
4-(1-methoxymethyl-propoxy)-2,5,6-trimethyl-7-(2,4,6-trimethylphenyl)-7H-pyrrolo[2,3-d]pyrimidine;
4-(1-ethyl-butyl)-2,5,6-trimethyl-7-(2,4,6-trimethylphenyl)-7H-pyrrolo-[2,3-d]pyrimidine;
[7-(4-bromo-2,6-dimethyl-phenyl)-2,5-dimethyl-7H-pyrrolo[2,3-d]pyrimidin-4-yl]-(1-methoxymethyl-propyl)-amine;
2-[7-(2-bromo-4,6-dimethyl-phenyl)-2,5-dimethyl-7H-pyrrolo[2,3-d]pyrimidin-4-ylamino]-butan-1-ol;
2-[7-(4-ethyl-2,6-dimethyl-phenyl)-2,5-dimethyl-7H-pyrrolo[2,3-d]pyrimidin-4-ylamino]-butan-1-ol;
2-[7-(2-ethyl-4,6-dimethylphenyl)-2,5-dimethyl-7H-pyrrolo[2,3-d]pyrimidin-4-ylamino]-butan-1-ol; and
2-[7-(2-fluoromethyl-4,6-dimethylphenyl)-2,5-dimethyl-7H-pyrrolo[2,3-d]pyrimidin-4-ylamino]-butan-1-ol.

16. The use according to claim 1 or claim 2, wherein the medicament is for treating or preventing a stress induced immune dysfunction in a livestock animal that is induced by the stress of confinement.
